# EUROPÄISCHE PATENTANMELDUNG

(11) **EP 4 238 589 A1**
(43) Veröffentlichungstag der Anmeldung: **06.09.2023**
(21) Anmeldenummer: 23159787.3
(22) Anmeldetag: 02.03.2023
(51) Int. Cl.: A61L 9/12

(54) **WIRKSTOFFKAPSEL ZUR SPEICHERUNG UND ABGABE VON VERDUNSTBAREN FLU SSIGEN WIRKSTOFFEN, INSBESONDERE AROMASUBSTANZEN**

(30) Priorität: 03.03.2022 DE 102022105100
(71) Anmelder: Schotte, Christopher, 45731 Waltrop (DE)
(72) Erfinder: Schotte, Christopher, 45731 Waltrop (DE)
(74) Vertreter: Tarvenkorn, Oliver

(57) **Zusammenfassung**

Wirkstoffkapsel (100) zur Speicherung und Abgabe von verdunstbaren flüssigen Wirkstoffen, insbesondere Aromasubstanzen (30), wenigstens umfassend:
- ein Unterteil (10) mit einem äußeren Bodenbereich (15) und einem diesen gegenüber verschiebbaren Zentralbereich (11);
- ein in oder an dem äußeren Bodenbereich (15) gelagertes, semipermeables Wirkstoffspenderelement (20) mit einer zylindrischen Innenwandung (21), die eine Ausnehmung (22) umgibt,
- ein Vorratskammerelement (40), das nach oben durch einen Deckelabschnitt (42) abgeschlossen ist und das eine Wandung (41) aufweist,
wobei:
- das Vorratskammerelement (40) nach unten offen ist;
- in einer Ausgangsstellung die Wandung (41) des Vorratskammerelement (40) gegenüber dem Unterteil (10) durch Eingriff in eine Ringnut (14) abgedichtet ist und zwischen dem Vorratskammerelement (40) und dem Unterteil (10) ein abgeschlossener Aufnahmeraum (50) ausgebildet ist; und
- in einer Wirkstoffabgabestellung das Vorratskammerelement (40) vom Unterteil (10) abgehoben ist und der Aufnahmeraum (50) zumindest teilweise zu der Innenwandung (21) des Wirkstoffspenderelements (20) hin geöffnet ist.

## Beschreibung

Die Erfindung betrifft eine Wirkstoffkapsel zur Speicherung und Abgabe von verdunstbaren flüssigen Wirkstoffen, insbesondere Aromasubstanzen mit den Merkmalen des Oberbegriffs des Anspruchs 1.

Es ist bekannt, Aromasubstanzen über Holzstäbe an die Raumluft abzugeben. Dafür wird eine Flasche mit einem engen Hals verwendet, in der die flüssige Aromasubstanz vorrätig gehalten wird. Nach dem Abnehmen des Flaschendeckels werden ein oder mehrere Holzstäbe in die Flasche gestellt, so dass aufgrund der Kapillarwirkung der Holzporen die flüssige Aromasubstanz im Holzstab aufsteigt und außerhalb der Flasche verdunstet. Die Flasche ist aber während der Duftabgabe offen und kann z. B. umkippen, so dass durch die oft öligen Aromasubstanzen Flecken im Umgebungsbereich verursacht werden. Außerdem benötigt die Anordnung der offenen Flasche mit den daraus herausragenden Holzstäben relativ viel Platz. Soll die Aromaabgabe beendet werden, muss der getrennt aufbewahrte Deckel wieder aufgeschraubt werden und die Holzstäbe müssen entsorgt werden. Für eine erneute Verwendung müssen neue Holzstäbe bereitgehalten werden.

Die WO 92/015338 A1 beschreibt ein becherförmiges Behältnis zur Abgabe von Aromasubstanzen an die Raumluft, das mit einem Deckel verschlossen ist, wodurch ein abgeschlossener Aufnahmeraum für die Aromasubstanz geschaffen ist. Außerdem ist ein poröses, ringförmiges Wirkstoffspenderelement vorgesehen, das einen Teil der im Behälter vorrätig gehaltenen Aromasubstanz speichern kann und aufgrund seiner Porosität eine zeitliche verzögerte Verdunstung ermöglicht, sobald der Deckel vom Behälter abgenommen ist und das Wirkstoffspenderelement frei liegt. Nachteilig hieran ist, dass der abgenommene Deckel zwischengelagert werden muss. Auch muss der Behälter von Zeit zu Zeit gekippt werden, um das im oberen Bereich gelagerte Wirkstoffspenderelement erneut mit der flüssigen Aromasubstanz zu tränken.

DE 20 2008 015 785 U1 beschreibt einen Duftspender zur Speicherung und Abgabe von verdunstbaren Aromasubstanzen, die in einem ausgehärteten Wachskörper inkorporiert sind, mit einem Gehäuse und einem Deckel, wobei der Boden des Gehäuses einen verschiebbaren Zentralbereich in Form eines Verstellmechanismus zur vertikalen Verschiebung des Wachskörpers aufweist, so dass dieser bei geöffnetem Gehäuse aus selbigen zur Abgabe der Duftstoffe heraus geschoben werden kann. Der Verstellmechanismus weist einen im Boden des Gehäuses gelagerten Drehknopf auf, der mit einer Gewindestange verbunden ist, die in ein Schraubgewinde eingreift, welches sich im Inneren des Wachskörpers befindet. Jedoch weist der Duftspender kein am äußeren Bodenbereich gelagertes, semipermeables Wirkstoffspenderelement auf, und zum anderen wird in der Wirkstoffabgabestellung nicht ein Vorratskammerelement vom Boden abgehoben, sondern der Duftwachskörper selbst. Im Übrigen ist der Duftspender auch nicht zur Abgabe von flüssigen Wirkstoffen geeignet.

Eine mehrteilige Beduftungsvorrichtung mit einem separaten Unterteil, das in seinem zentralen Bereich ein rotierendes Element besitzt, ist aus CN 212 827 805 U bekannt. Das Vorratskammerelement besitzt eine Wandung, die nach oben durch einen Deckelabschnitt abgeschlossen und nach unten offen ist.

Das Vorratskammerelement ist zur Aufnahme eines Duftstoffträgers vorgesehen, wobei ein Duftstoffträger auch mit einem flüssigen Duftstoff getränkt sein kann. Die Wirk-/Duftstoffabgabe wird dadurch gesteuert, dass mittels des Verstellmechanismus Öffnungen zwischen einer Wirkstoffvorratskammer und einem Deckel eingestellt werden. Damit können aber weiterhin flüssige Duft- oder Wirkstoffe auslaufen, wenn die Beduftungsvorrichtung umkippt.

Bei einer in CN 209 286 257 U gezeigten Beduftungsvorrichtung ist ein torusförmiges Außenteil vorgesehen. Im Inneren ist eine Kammer gebildet, die durch einen Deckel abgeschlossen ist. Der Deckel wird über ein Schraubgewinde beim Drehen angehoben, so dass auch hier Öffnungen zwischen einer Wirkstoffvorratskammer und einem Deckel eingestellt werden, die das Auslaufen von Flüssigkeiten nicht verhindern. In gleicher Weise funktionieren jeweils die in CN 211 157 526 U und CN 211 000 853 U beschriebenen Beduftungsvorrichtungen, wobei die Art und Weise, wie der Deckel gegenüber einer Wandung einer Vorratskammer angehoben wird, jeweils unterschiedlich ist.

Die Aufgabe der Erfindung besteht somit darin, eine platzsparende Wirkstoffkapsel anzugeben, in der Flüssigkeiten speicherbar sind, die dann nach Benutzer-Aktivierung langsam verdunsten können und an die Raumluft abgegeben werden, ohne dass zur Aktivierung Teile abgenommen werden müssen.

Diese Aufgabe wird durch eine Wirkstoffkapsel mit den Merkmalen des Anspruchs 1 gelöst.

Die Wirkstoffkapsel nach der Erfindung ermöglicht einen problemlosen Wechsel zwischen einer Ausgangsstellung für den Transport und die Lagerung, in der keine Abgabe von Wirkstoffen stattfindet, und einer Duft- oder Wirkstoffabgabestellung, in der flüssige Aromasubstanzen verdunsten können.

Als Wirkstoffe kommen insbesondere Duftstoffe in Betracht, um Aromasubstanzen an die Raumluft abzugeben. Möglich sind auch andere flüssige Mittel, für die eine langsame, niedrig dosierte Freisetzung an die Umgebung gewünscht wird wie beispielsweise Mittel zur Schädlingsbekämpfung im Haushalt.

Bei der Wirkstoffkapsel nach der Erfindung bildet ein Wirkstoffspenderelement, über welches die Verdunstung einer im Inneren freigesetzten, flüssigen Wirkstoffsubstanz erfolgt, zugleich die Gehäuseaußenseite, so dass eine große Oberfläche für die Verdunstung der aufgenommenen Wirkstoffe zur Verfügung steht. Besonders vorteilhaft ist eine Torusform, also eine Ringform mit rundem oder gerundetem Querschnitt, da hierbei bezogen auf die Abmaße eine maximale Oberfläche zur Verfügung steht.

Das Wirkstoffspenderelement besteht vorzugsweise aus einem semipermeablen Werkstoff wie insbesondere Zedernholz oder Sandelholz. Die Porenstruktur derartiger Hölzer ist semipermeabel in dem Sinne, dass einerseits das Auslaufen einer flüssigen oder gelartigen Aromasubstanz verhindert wird, dass aber andererseits eine langsame Migration der Aromasubstanz oder eines anderen Wirkstoffs durch den Holzkörper hindurch bis an die Außenseite des Wirkstoffspenderelements möglich ist, wo die Aromen oder andere Wirkstoffe an die Raumluft abgegeben werden.

Außer Naturholz sind auch Wirkstoffspenderelemente einsetzbar, die aus gepressten Holzschnitzeln, Sägemehl, Papierresten, Zellstoff oder Naturfasern wie Baumwolle, Hanf, Flachs, Schilf oder Bambus bestehen.

Die gespeicherten und nach Bedarf freizusetzenden Wirkstoffe müssen insbesondere hinsichtlich ihrer Viskosität so beschaffen sein und auf die Porenstruktur des Wirkstoffspenderelements abgestimmt sein, dass sie von diesem problemlos aufgesaugt werden können und an dessen Außenseite migrieren können. Auch muss das Lösemittel der Aromasubstanz an der Raumluft bei üblichen Temperaturen verdunstbar sein. Das Lösemittel kann Wasser und/oder ein organisches Lösemittel wie insbesondere ein Alkohol sein. Der Durchgang des Wirkstoffs durch das Wirkstoffspenderelement soll mit einer solchen zeitlichen Verzögerung geschehen, dass die migrierte Flüssigkeit vollständig verdunsten kann, ohne dass sie außen am Wirkstoffspenderelement abtropft.

Erfindungswesentlich ist, dass mit nur zwei zusätzlich zum Wirkstoffspenderelement vorgesehen Bauteilen ein dichter Aufnahmeraum geschaffen werden kann, in welchem die Aromasubstanz oder ein anderer Wirkstoff aufbewahrt wird. Um die Duftabgabe einzuleiten, werden die beiden Bauteile, nämlich ein als Basis dienendes Unterteil und ein Vorratskammerelement, voneinander entfernt, so dass sich der dazwischen ausgebildete Aufnahmeraum am Außenumfang öffnet und die Aromasubstanz an die Innenwandung des Wirkstoffspenderelements gelangt. Sie migriert dann durch den Körper des Wirkstoffspenderelements hindurch bis an dessen äußere Oberfläche und verdunstet dort.

Die Wirkstoffkapsel ist üblicherweise zum einmaligen Gebrauch vorgesehen, das heißt, eine im Vorratsraum bevorratete flüssige Substanz wird vom Benutzer durch Tastendruck freigesetzt und wird vollständig vom Wirkstoffspenderelement aufgesaugt.

Soll die Wirkstoffabgabe gestoppt werden, kann vorgesehen sein, dass der Aufnahmeraum wieder geschlossen werden kann, so dass nur noch die Reste der Aromasubstanz, die mit dem Wirkstoffspenderelement in Kontakt sind, verdunsten, während der restliche Teil der Aromasubstanz wieder im abgedichteten Aufnahmeraum aufbewahrt wird.

Besonders vorteilhaft ist die Art und Weise, wie nach der Erfindung der Aufnahmeraum geöffnet wird. Das Vorratskammerelement ist innerhalb einer zentralen Ausnehmung im Wirkstoffspenderelement verschiebbar angeordnet. Das Vorratskammerelement ist an seiner Unterseite hohl und dichtet mit der Unterkante seiner Wandung gegenüber dem Boden am Unterteil ab, wenn es sich in einer abgesenkten Stellung befindet. Wird es angehoben, wird die Dichtung aufgehoben, und die Aromasubstanz oder der Wirkstoff kommt in direkten Kontakt mit der Innenwandung des Wirkstoffspenderelements.

Ein weiterer Vorteil besteht darin, dass der Boden flexibel ausgestaltet ist und formschlüssig mit dem Vorratskammerelement verbunden ist, so dass ein Zentralbereich am Boden mit dem Vorratskammerelement zusammen angehoben wird, wenn der Aufnahmeraum geöffnet wird. Die erhöhte Stellung des Zentralbereichs in der Wirkstoffabgabestellung hat den weiteren Vorteil, dass keine Flüssigkeitsreste in Vertiefungen am Boden verbleiben, sondern vielmehr von einer erhöhten Stellung des Bodens aus radial zu dem außen gelegenen Wirkstoffspenderelement abfließen.

Zur Verschiebung des Zentralbereichs des Bodens bei gleichzeitiger Aufrechterhaltung einer dichten Bodenstruktur ist vorgesehen, dass der äußere Bodenbereich des Unterteils und der Zentralbereich über wenigstens ein ringförmiges Filmscharnier und/oder eine elastische Dehnungszone miteinander verbunden sind.

Dazu kann auch vorgesehen sein, einen Bodenbereich aus einem harten Werkstoff für den äußeren Bodenbereich mit einem elastomeren Werkstoff für den Zentralbereich zu kombinieren.

Eine vorteilhafte Ausführungsform sieht vor, dass zur Abdichtung zwischen dem Vorratskammerelement und dem Boden eine bodenseitige Ringnut vorgesehen ist, in die die Unterkante der zylindrischen Wandung eingreift. Auch dazu ist es vorteilhaft, wenn der Bereich, an dem die Ringnut ausgebildet ist, aus einem elastomeren Werkstoff gebildet ist.

Da der Aufnahmeraum in der Ausgangsstellung allseitig abgedichtet ist, kann keine Aromaflüssigkeit auslaufen. Die Wirkstoffkapsel kann problemlos transportiert werden, wobei keine besondere Transportlage eingehalten zu werden braucht. Es muss lediglich darauf geachtet werden, dass bei Transport und Lagerung kein Druck auf den Zentralbereich des Unterteils ausgeübt wird. Bevorzugt sind die Unterkante des Vorratskammerelements und die Ringnut am Boden und/oder ein innerhalb des Vorratskammerelements angeformter Steg und eine zugehörige Aufnahmenut am Boden so profiliert, dass dazwischen eine Rastverbindung ausgebildet ist und die Verschiebung zwecks Aktivierung der Wirkstoffkapsel nur bewusst und mit einer größeren Kraft möglich ist, so dass eine versehentliche Aktivierung vermieden wird.

Das Vorratskammerelement besitzt vorzugsweise eine zylindrische Wandung, die in eine kreisrunde Ringnut am Unterteil eingreift, und auch die Innenwandung des Wirkstoffspenderelements ist bevorzugt zylindrisch. Bei allen drei genannten Elementen sind aber auch andere aufeinander abgestimmte Grundrissformen möglich, z. B. polygonale Strukturen. Die Elemente müssen so abgestimmt sein, dass das Vorratskammerelement innerhalb der Ausnehmung des Wirkstoffspenderelements ungehindert verschiebbar ist und die Wandung des Vorratskammerelement so in die Ringnut am Boden eingreifen kann, dass dazwischen eine Abdichtung gewährleistet ist.

Die Erfindung wird nachfolgend mit Bezug auf das in den Zeichnungen dargestellte Ausführungsbeispiel näher erläutert. Die Figuren zeigen jeweils in perspektivischer Ansicht:
- Fig. 1: eine Wirkstoffkapsel nach einer ersten Ausführungsform der Erfindung in einer Explosionsdarstellung;
- Fig. 2: die Wirkstoffkapsel in einer Ausgangsstellung in Ansicht von außen;
- Fig. 3: die Wirkstoffkapsel in der Ausgangsstellung in Schnittansicht;
- Fig. 4: die Wirkstoffkapsel in einer Wirkstoffabgabestellung in Ansicht von außen;
- Fig. 5: die Wirkstoffkapsel in der Wirkstoffabgabestellung in Schnittansicht;
- Fig. 6: eine Wirkstoffkapsel nach einer zweiten Ausführungsform der Erfindung in einer Explosionsdarstellung;
- Fig. 7: die Wirkstoffkapsel nach Figur 6 in der Ausgangsstellung in Schnittansicht;
- Fig. 8: eine Vergrößerung aus Figur 7 und
- Fig. 9: die Wirkstoffkapsel nach Figur 6 in der Wirkstoffabgabestellung in Schnittansicht.

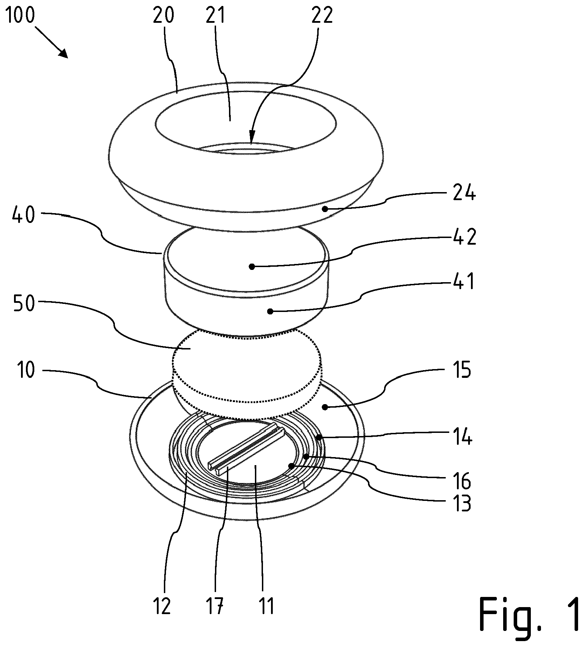

In Figur 1 ist eine Wirkstoffkapsel 100 nach der Erfindung in einer Explosionsdarstellung dargestellt. Diese besteht im Wesentlichen aus einem Unterteil 10, einem Wirkstoffspenderelement 20 und einem Vorratskammerelement 40. Dazwischen ist ein mit punktierten Linien angedeuteter Aufnahmeraum 50 ausgebildet, der mit einem flüssigen Wirkstoff wie insbesondere einer aromatischen Substanz gefüllt werden kann.

Das Unterteil 10 hat im dargestellten Ausführungsbeispiel eine runde Außenkontur. Es besteht aus einem flüssigkeitsdichten Werkstoff und unterteilt sich in einen ringförmigen äußeren Bodenbereich 15 und einen Zentralbereich 11. Diese sind über eine Dehnungszone 12 miteinander verbunden, welche so ausgebildet ist, dass der Zentralbereich 11 relativ zu dem äußeren Bodenbereich 15 verschoben werden kann, wobei eine flüssigkeitsdichte Bodenstruktur erhalten bleibt. Dazu sind in der Dehnungszone 12 zwei konzentrische, kreisringförmige Filmscharniere 13, 16 vorgesehen. Im äußeren Bodenbereich 15 sind außerdem zwei konzentrische Stege ausgebildet, die zwischen sich eine Ringnut 14 ausbilden.

Das Wirkstoffspenderelement 20 ist im gezeigten Beispiel annähernd torusförmig. Es liegt mit einem Basisabschnitt 24 auf dem schüsselartig geformten äußeren Bodenbereich 15 des Unterteils 10 auf. Der Innendurchmesser einer zentralen Ausnehmung 22 bzw. der zylindrischen Innenwandung 21, die die Ausnehmung 22 begrenzt, ist so gewählt, dass die Filmscharniere 13, 16 und die Ringnut 14 innerhalb der Ausnehmung 22 liegen.

Das Vorratskammerelement 40 ist ein zylindrischer Becher mit einem Deckelabschnitt 42 und einer zylindrischen Wandung 41. Es ist innen hohl und nach unten offen. Das Vorratskammerelement 40 besteht, wie das Unterteil 10 auch, aus einem flüssigkeitsdichten Werkstoff wie einem thermoplastischen Kunststoff oder Aluminium, so dass aromatische Substanzen nicht hindurch migrieren können.

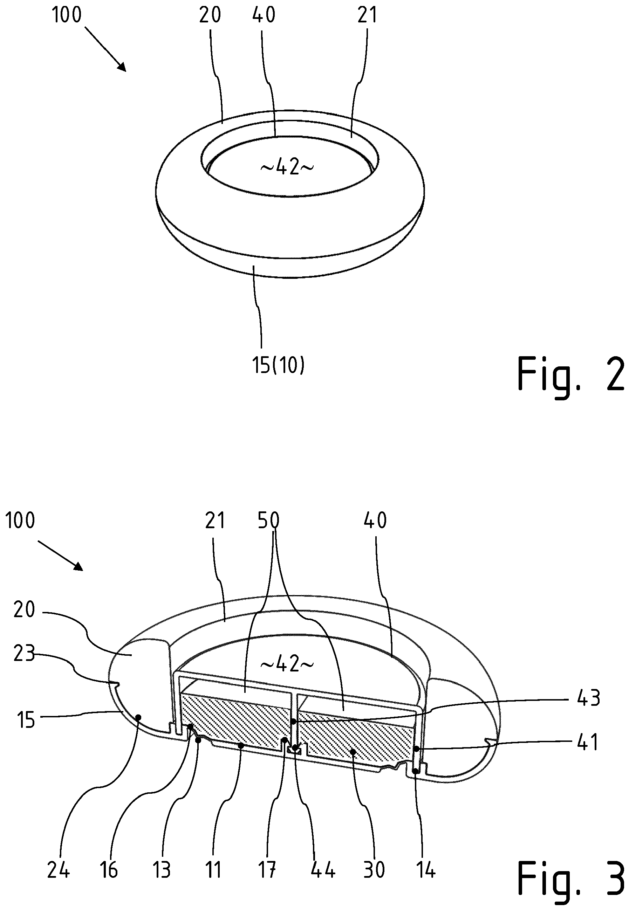

Fig. 2 zeigt die Wirkstoffkapsel 100 in einer Ausgangsstellung in Ansicht von außen. Das Wirkstoffspenderelement 20 aus Holz ist mit dem Unterteil 10 fest verbunden. Von dem Vorratskammerelement 40 ist nur der Deckelabschnitt 42 sichtbar, der etwas vertieft liegt.

Fig. 3 zeigt einen Schnitt durch die Wirkstoffkapsel 100 in der Ausgangsstellung. Die Verbindung zwischen dem Wirkstoffspenderelement 20 und dem Unterteil 10 erfolgt über eine Rastverbindung. Dazu besitzt das Wirkstoffspenderelement 20 eine Rastnut 23 am Außenumfang, in die ein Profilvorsprung am Außenrand des äußeren Bodenbereichs 15 eingreift. Am Boden des Unterteils 10 befindet sich der Zentralbereich 11 auf gleicher Höhe wie der benachbarte Teil des äußeren Bodenbereichs 15.

Das Vorratskammerelement 40 besitzt einen an die Unterseite des Deckelabschnitts 42 angeformten Steg 43 mit einem endseitigen Ankerfuß 44. Steg 43 und Ankerfuß 44 bilden ein Koppelelement, über welches das Vorratskammerelement 40 formschlüssig mit dem Zentralbereich 11 des Unterteils 10 verbunden ist. Dazu rastet der Ankerfuß 44 in die Nut 17 ein. Zwischen dem Zentralbereich 11 und dem äußeren Bodenbereich 15 ist die Wandstärke des Unterteils 10 reduziert. Dort sind auch die Filmscharniere 13, 16 vorgesehen. Dadurch ist dieser Bereich dehnbar.

Zwischen dem Zentralbereich 15 und dem damit gekoppelten Vorratskammerelement 40 ist ein Aufnahmeraum 50 ausgebildet. Der dort hineinragende Steg 43 erstreckt sich nicht über den gesamten Durchmesser, so dass der Steg 43 den Aufnahmeraum 50 nicht in abgeschlossene Bereiche unterteilt. Die in dem Aufnahmeraum 50 vorrätig gehaltene Aromasubstanz 30 kann sich somit gleichmäßig darin verteilen.

Der Aufnahmeraum 50 ist in der gezeigten Stellung allseitig geschlossen. Sein Volumen ist durch den Zentralbereich 11 am Boden und das becherförmige Vorratskammerelement 40 definiert. Eine Dichtigkeit des Aufnahmeraums 50 wird dadurch erreicht, dass die zylindrische Wandung 41 des Vorratskammerelements 40 mit ihrer Kante in die Ringnut 14 am Boden des Unterteils 10 eingreift.

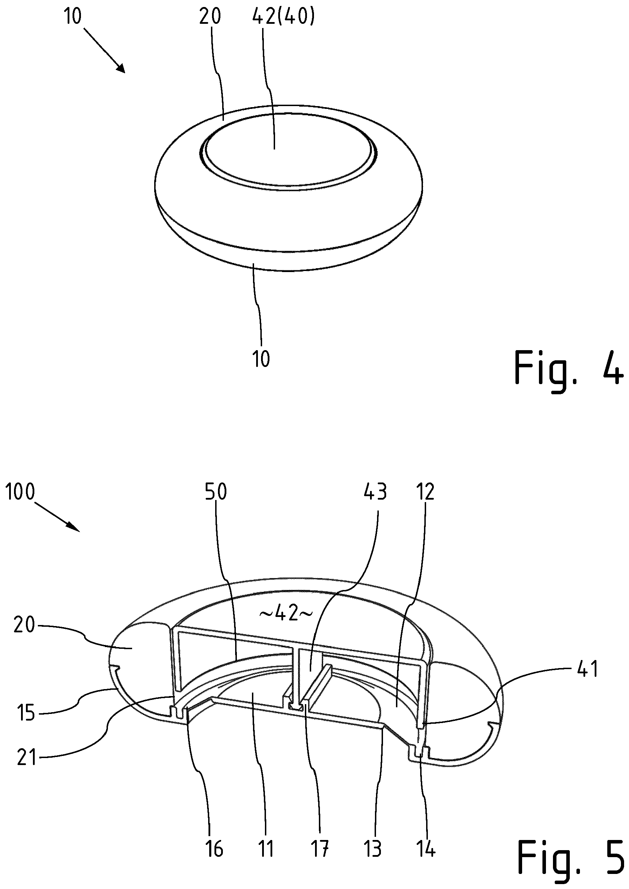

Um nun Aromen von der Wirkstoffkapsel 100 an die Raumluft abgeben zu können, wird der Zentralbereich 11 von der Unterseite her nach oben gedrückt. Dadurch wird auch das gekoppelte Vorratskammerelement 40 mit dem Deckelbereich 42 angehoben, wie in der Darstellung der Wirkstoffabgabestellung in Figur 4 gezeigt.

Figur 5 zeigt die Wirkstoffkapsel 100 in der Wirkstoffabgabestellung im Schnitt. Die Wirkstoffkapsel 100 muss in der abgebildeten Lage positioniert sein, das heißt, das Unterteil 10 liegt auf einem festen Gegenstand wie einem Tisch oder Regalbrett auf.

Der Zentralbereich 11 ist jetzt gegenüber der Unterseite des Unterteils 15 erhöht angeordnet. Zugleich bleibt der Bodenbereich aber dicht. Dies ist dadurch ermöglicht, dass zwei Filmscharniere 13, 16 vorgesehen sind, die das Einstülpen des Zentralbereichs 11 erlauben.

Mit der Verschiebung des Zentralbereichs 11 wird auch das gekoppelte Vorratskammerelement 40 verschoben worden, wobei es mit der Kante seiner zylindrischen Wandung 41 aus der Ringnut 14 ausgerückt ist. Dadurch wird die Dichtwirkung an der Bodeninnenseite aufgehoben. Der Aufnahmeraum 50 wird zugleich seitlich etwas geöffnet, so dass die im Aufnahmeraum 50 gespeicherte Aromasubstanz in direkten Kontakt mit der Innenwand 21 des Wirkstoffspenderelements 20 gelangt. Durch eine langsame Migration der Aromasubstanz durch das Porensystem des insbesondere aus Holz bestehenden Wirkstoffspenderelements 20 gelangen Teile der Aromasubstanz an die Außenseite des Wirkstoffspenderelements 20 und verdunsten dort, so dass die Moleküle an die Raumluft abgegeben werden können.

Da der Zentralbereich 11 des Bodens angehoben ist und der Bereich zwischen den Filmscharnieren 13, 16 eine schiefe Ebene bildet, kann Restflüssigkeit gut radial nach außen abfließen bis der Aufnahmeraum 50 vollkommen entleert ist.

Der Übergang von der Speicherstellung in die Wirkstoffabgabestellung kann reversibel sein. Indem das Vorratskammerelement 40 durch Druck auf seinen Deckelbereich 42 gegenüber dem Wirkstoffspenderelement 20 nach unten gedrückt wird, wird die Einstülpung des Zentralbereichs 11 des Bodens an den Filmscharnieren 13, 16 wieder rückgängig gemacht. Außerdem wird die Kante der zylindrischen Wandung 41 wieder in die Ringnut 14 eingeführt, so dass der unter dem Vorratskammerelement 40 ausgebildete Aufnahmeraum 50 wieder nach allen Seiten abgeschlossen ist und die Stellung der Teile gemäß Figur 3 wiederhergestellt ist.

Sobald die noch in den Poren des Wirkstoffspenderelements 20 vorhandene Restflüssigkeit vollständig verdunstet ist, erfolgt keine Duft- oder Wirkstoffabgabe an die Raumluft mehr. Die Wirkstoffkapsel 100 kann in der Ausgangsstellung gut gelagert und transportiert werden.

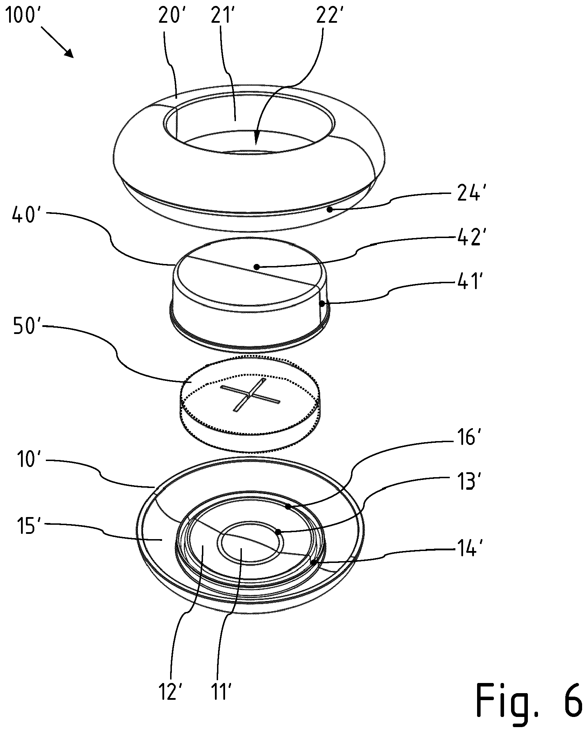

In Figur 6 ist eine Wirkstoffkapsel 100' nach einer zweiten Ausführungsform der Erfindung in einer Explosionsdarstellung dargestellt. Diese besteht wiederum aus einem Unterteil 10', einem Vorratskammerelement 40' und einem zur ersten Ausführungsform nahezu identischen Wirkstoffspenderelement 20'. Dazwischen ist ein mit punktierten Linien angedeuteter Aufnahmeraum 50' ausgebildet, der mit einem Wirkstoff wie insbesondere einer aromatischen Substanz gefüllt werden kann. Die Kreuzstruktur in der Mitte kennzeichnet einen nicht sichtbaren Steg, der im Zentrum des Vorratskammerelement 40' angeformt ist

Das Unterteil 10' ist rund und besteht aus einem flüssigkeitsdichten Werkstoff. Es unterteilt sich in einen ringförmigen äußeren Bodenbereich 15', eine Dehnungszone 12' und einen Zentralbereich 11'. Am Übergang zwischen der Dehnungszone 12' und dem äußeren Bodenbereich 15' sind zwei konzentrische Stege ausgebildet, die zwischen sich eine Ringnut 14' ausbilden.

Die Dehnungszone 12' ist so ausgebildet ist, dass der Zentralbereich 11' relativ zu dem äußeren Bodenbereich 15' verschoben werden kann, wobei eine flüssigkeitsdichte Bodenstruktur erhalten bleibt. Dazu sind in der Dehnungszone 12' zwei konzentrische, kreisringförmige Filmscharniere 13', 16' vorgesehen.

Das Vorratskammerelement 40' ist ein zylindrischer Becher mit einem Deckelabschnitt 42' und einer zylindrischen Wandung 41'. Es ist innen hohl und nach unten offen. Das Vorratskammerelement 40' besteht, wie das Unterteil 10' auch, aus einem flüssigkeitsdichten Werkstoff wie einem thermoplastischen Kunststoff oder Aluminium, so dass aromatische Substanzen nicht hindurch migrieren können.

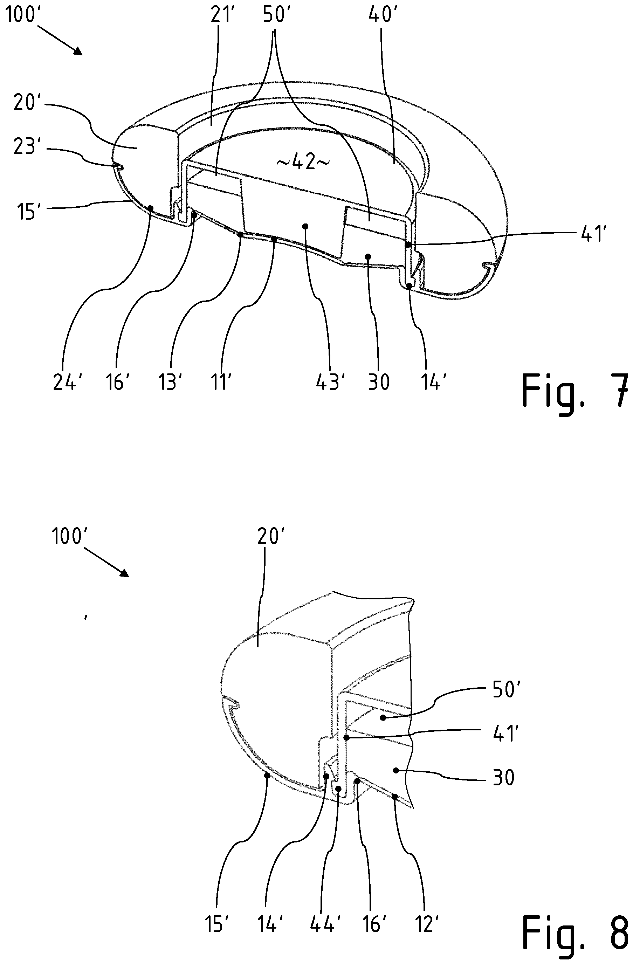

Figur 7 ist eine Schnittansicht der Speicher- oder Ausgangsstellung mit einer in einem Aufnahmeraum 50' gespeicherten Aromasubstanz.

Die Verbindung zwischen dem Wirkstoffspenderelement 20` und dem Unterteil 10' erfolgt über eine Rastverbindung. Dazu besitzt das Wirkstoffspenderelement 20' eine Rastnut 23' am Außenumfang, in die ein Profilvorsprung am Außenrand des äußeren Bodenbereichs 15' eingreift. Am Boden des Unterteils 10' befindet sich der Zentralbereich 11' auf gleicher Höhe wie der benachbarte Teil des äußeren Bodenbereichs 15'.

Zwischen dem Zentralbereich 11' und dem äußeren Bodenbereich 15 ist eine Dehnungszone 12' ausgebildet, in der die Wandstärke des Unterteils 10' reduziert ist. Zwischen dem äußeren Bodenbereich 15' und der Dehnungszone 12' sowie zwischen letzterer und dem Zentralbereich 11' ist jeweils ein Filmscharnier 13', 16' vorgesehen.

Zwischen dem Zentralbereich 11' und dem damit gekoppelten Vorratskammerelement 40' ist der Aufnahmeraum 50' ausgebildet, der in der gezeigten Stellung allseitig geschlossen ist. Sein Volumen ist durch den Zentralbereich 11' am Unterteil 10' und das becherförmige Vorratskammerelement 40' definiert. Eine Dichtigkeit des Aufnahmeraums 50' wird dadurch erreicht, dass die zylindrische Wandung 41' des Vorratskammerelements 40' mit ihrer Kante in die Ringnut 14' am Boden des Unterteils 10' eingreift.

Das Vorratskammerelement 40' besitzt neben der zylindrischen Wandung 41' am Außenumfang einen an die Unterseite des Deckelabschnitts 42' angeformten, kreuzförmigen Steg 43' im Zentrum. Bei der Wirkstoffkapsel 100' ist die zylindrische Wandung 41' am unteren Ende mit einem Ankerfuß 44' versehen. Der Ankerfuß 44' bildet zusammen mit einer entsprechend profilierten Ringnut 14' ein Koppelelement, über welches das Vorratskammerelement 40' formschlüssig mit dem Zentralbereich 11' des Unterteils 10' verbunden ist. Dazu rastet der Ankerfuß 44' in die Ringnut 14'ein.

Die Rastverbindung zwischen der zylindrischen Wandung 41' und der aus zwei konzentrischen Stegen gebildeten Ringnut ist in Figur 8 in einer ausschnittsweisen Vergrößerung der Schnittdarstellung aus Figur 7 gezeigt. Der äußere Steg an der Ringnut 14' ist mit einem Hinterschnitt profiliert, so dass der Ankerfuß 44' dort einrasten kann.

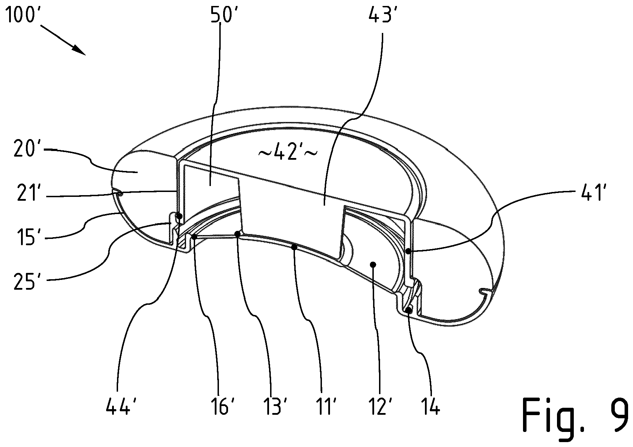

Um nun Aromen von der Wirkstoffkapsel 100' an die Raumluft abgeben zu können, wird der Zentralbereich 11' von der Unterseite her nach oben gedrückt. Dadurch wird Druck auf den Steg 43' ausgeübt und dadurch das Vorratskammerelement 40' mit dem Deckelbereich 42' angehoben, wie in der Darstellung der Wirkstoffabgabestellung in Figur 9 gezeigt.

Der Zentralbereich 11' ist jetzt gegenüber der Unterseite des Unterteils 15' erhöht angeordnet. Zugleich bleibt der Bodenbereich aber dicht. Dies ist dadurch ermöglicht, dass eine Dehnungszone 12' und zwei Filmscharniere 13', 16' vorgesehen sind, die das Einstülpen des Zentralbereichs 11' erlauben.

Mit der Verschiebung des Zentralbereichs 11' rückt das Vorratskammerelement 40' mit der zylindrischen Wandung 41' aus der Ringnut 14' aus. Dadurch wird die Dichtwirkung an der Bodeninnenseite aufgehoben. Der Aufnahmeraum 50' wird zugleich seitlich etwas geöffnet, so dass die im Aufnahmeraum 50' gespeicherte Aromasubstanz in direkten Kontakt mit der Innenwand 21' des Wirkstoffspenderelements 20' gelangt. Durch eine langsame Migration der Aromasubstanz durch das Porensystem des insbesondere aus Holz bestehenden Wirkstoffspenderelements 20' gelangen Teile der Aromasubstanz an die Außenseite des Wirkstoffspenderelements 20' und verdunsten dort, so dass die Moleküle an die Raumluft abgegeben werden können.

Da der Zentralbereich 11' des Bodens angehoben ist und die Dehnungszone 12' zwischen den Filmscharnieren 13', 16' eine schiefe Ebene bildet, kann Restflüssigkeit gut radial nach außen abfließen, bis der Aufnahmeraum 50' vollkommen entleert ist.

Der axiale Weg des Vorratskammerelements 40' gegenüber dem Wirkstoffspenderelement 20' ist durch einen Rücksprung 25' in der Innenwandung 21' begrenzt. Der Ankerfuß 44' greift am Ende der zur Verschiebung vorgesehenen Strecke unter die durch den Rücksprung 25' gebildete Kante und ist daran festgelegt. Andererseits ist die Dehnungszone 12' nach der Einstülpung des Zentralbereichs 11' elastisch vorgespannt, so dass das der Kontakt zwischen dem Zentralbereich 11' und dem Steg 43' gehalten wird und das Vorratskammerelement 40' nicht mehr nach unten sacken kann.

### Bezuaszeichen:

- 100; 100': Wirkstoffkapsel

- 10; 10': Unterteil
- 11; 11': Zentralbereich
- 12; 12': Dehnungszone
- 13; 13': ringförmiges Filmscharnier
- 14; 14': Ringnut
- 15; 15': äußerer Bodenbereich
- 16; 16': ringförmiges Filmscharnier
- 17: hinterschnittene Nut

- 20; 20': semipermeables Wirkstoffspenderelement
- 21; 21': zylindrische Innenwandung
- 22; 22`: Ausnehmung
- 23; 23': Rastnut
- 24; 24': Basisabschnitt
- 25': Rücksprung

- 30: Aromasubstanz

- 40; 40': Vorratskammerelement
- 41; 41': zylindrische Wandung
- 42; 42': Deckelabschnitt
- 43; 43': Steg
- 44; 44': Ankerfuß

- 50; 50`: Aufnahmeraum

## Patentansprüche

1. Wirkstoffkapsel (100; 100') zur Speicherung und Abgabe von verdunstbaren flüssigen Wirkstoffen, insbesondere Aromasubstanzen (30), wenigstens umfassend:
- ein Unterteil (10; 10') mit einem äußeren Bodenbereich (15; 15') und einem diesen gegenüber verschiebbaren Zentralbereich (11; 11');
- ein in oder an dem äußeren Bodenbereich (15; 15') gelagertes, semipermeables Wirkstoffspenderelement (20; 20') mit einer zylindrischen Innenwandung (21; 21'), die eine Ausnehmung (22; 22') umgibt,
- ein Vorratskammerelement (40; 40'), das nach oben durch einen Deckelabschnitt (42; 42') abgeschlossen und eine Wandung (41; 41') aufweist,
**dadurch gekennzeichnet:**
- **dass** das Vorratskammerelement (40; 40') nach unten offen ist;
- **dass** in einer Ausgangsstellung die Wandung (41; 41') des Vorratskammerelements (40; 40') gegenüber dem Unterteil (10; 10') durch Eingriff in eine Ringnut (14; 14') abgedichtet ist und zwischen dem Vorratskammerelement (40; 40') und dem Unterteil (10; 10') ein abgeschlossener Aufnahmeraum (50; 50') ausgebildet ist; und
- **dass** in einer Wirkstoffabgabestellung das Vorratskammerelement (40; 40') vom Unterteil (10; 10') abgehoben ist und der Aufnahmeraum (50; 50') zumindest teilweise zu der Innenwandung (21; 21') des Wirkstoffspenderelements (20; 20') hin geöffnet ist.

2. Wirkstoffkapsel (100; 100') nach Anspruch 1, **dadurch gekennzeichnet, dass** die Innenwandung (21; 21') des Wirkstoffspenderelements (20; 20'), die Wandung (41; 41') des Vorratskammerelements (40; 40') und die Ringnut (14, 14') jeweils zylindrisch sind und aufeinander abgestimmte Durchmesser besitzen.

3. Wirkstoffkapsel (100; 100') nach Anspruch 1 oder 2, **dadurch gekennzeichnet, dass** das Vorratskammerelement (40; 40') über wenigstens ein Koppelelement mit dem Zentralbereich (11; 11') des Unterteils (10; 10') verrastet ist.

4. Wirkstoffkapsel (100) nach Anspruch 3, **dadurch gekennzeichnet, dass** das Koppelelement durch einen an eine Unterseite des Deckelabschnitts (42) angeformten Steg (43) mit einem endseitigen Ankerfuß (44) gebildet ist und dass der Ankerfuß (44) in eine hinterschnittene Nut (17) im Zentralbereich (11) eingerastet ist.

5. Wirkstoffkapsel (100') nach Anspruch 3, **dadurch gekennzeichnet, dass** das Koppelelement durch die zylindrische Wandung (41') mit einem endseitigen Ankerfuß (44') gebildet ist und dass der Ankerfuß (44') in die Ringnut (14`), die im Querschnitt wenigstens einseitig hinterschnitten ist, eingerastet ist.

6. Wirkstoffkapsel (100') nach Anspruch 5, **dadurch gekennzeichnet, dass** das Vorratskammerelement (40') einen an eine Unterseite des Deckelabschnitts (42') angeformten Steg (43') aufweist, der beim Verschieben des Vorratskammerelements (40') vom Ausgangszustand in eine Wirkstoffabgabestellung den Zentralbereich (11') kontaktiert.

7. Wirkstoffkapsel (100; 100') nach einem der vorhergehenden Ansprüche, **dadurch gekennzeichnet, dass** der äußere Bodenbereich (15; 15') und der Zentralbereich (11; 11') über wenigstens ein ringförmiges Filmscharnier (13, 16; 13', 16') und/oder über eine elastische Dehnungszone (12; 12') miteinander verbunden sind.

8. Wirkstoffkapsel (100; 100') nach einem der vorhergehenden Ansprüche, **dadurch gekennzeichnet, dass** das Wirkstoffspenderelement (20; 20') an seinem Außenumfang eine Rastnut (23; 23') aufweist und dass das Unterteil (10; 10') einen nach innen gerichteten Rastvorsprung aufweist, der in die Rastnut (23; 23') eingreift.

9. Wirkstoffkapsel (100; 100') nach einem der vorhergehenden Ansprüche, **dadurch gekennzeichnet, dass** die zylindrische Wandung (41; 41') des Vorratskammerelements (40; 40') an der Innenwandung (21; 21') des Wirkstoffspenderelements (20; 20') geführt ist.

10. Wirkstoffkapsel (100; 100') nach einem der vorhergehenden Ansprüche, **dadurch gekennzeichnet, dass** das Vorratskammerelement (40; 40') nach oben durch einen ebenen Deckelabschnitt (42; 42') abgeschlossen ist, der in der Wirkstoffabgabestellung auf Höhe der Oberkante des Wirkstoffspenderelements (20; 20') angeordnet ist und in der Ausgangsstellung vertieft dieser gegenüber in der Ausnehmung (22; 22') angeordnet ist.
